# FASCICULE DE BREVET EUROPEEN

(11) **EP 1 162 950 B1**
(45) Date de publication et mention de la délivrance du brevet: **10.10.2007**
(21) Numéro de dépôt: 00910955.4
(22) Date de dépôt: 16.03.2000
(51) Int. Cl.: A61K 9/127

(54) **MILIEUX SOUS FORME DE DISPERSIONS COMPLEXES, LEUR PROCEDE DE PREPARATION ET LEURS UTILISATIONS**
MITTEL IN FORM EINER KOMPLEXDISPERSION, VERFAHREN ZU IHRER HERSTELLUNG UND IHRE VERWENDUNG
MEDIA IN THE FORM OF COMPLEX DISPERSIONS, METHOD FOR PREPARING SAME AND USES

(30) Priorité: 16.03.1999 FR 9903201
(43) Date de publication de la demande: 19.12.2001
(73) Titulaire: CAPSULIS, 33600 Pessac (FR)
(72) Inventeur: Degert, Corinne, F-33160 Saint Médard-en-Jalles (FR); Poulin, Philippe, Rés. Charles François Daubigny, 33400 Talence (FR); Ugazio, Stéphane, Bebington, Wirral CH633BU (GB); Laversanne, René, F-33600 Pessac (FR); Roux, Didier, F-33700 Mérignac (FR)
(74) Mandataire: Giraud, Françoise
(86) Numéro de dépôt international: PCT/FR2000/000631
(87) Numéro de publication internationale: WO 2000/054749

(56) Documents cités:
- WO-A-98/02144
- US-A- 5 256 422

## Description

La présente invention concerne de nouveaux milieux sous forme de dispersions complexes, leur procédé de préparation et leurs utilisations.

Différentes approches ont été utilisées pour libérer progressivement des actifs en recourant à des milieux structurés. Les émulsions sont les milieux structurés les plus simples, avec des compartiments par exemple huileux, dispersés dans un milieu continu aqueux (émulsion dite directe ou H/E, le contraire, eau dans huile étant appelé émulsion inverse ou E/H). Cependant ces systèmes, s'ils sont largement utilisés ne permettent pas de séparer deux milieux de même nature, comme un soluté hydrophile que l'on voudrait disperser dans un milieu continu aqueux, en empêchant sa solubilisation dans ce milieu continu.

Cet inconvénient a été contourné par l'utilisation d'émulsions multiples, consistant en une première émulsion, par exemple inverse E₁/H, de gouttelettes d'eau dans un milieu huileux, elle-même émulsionnée dans un milieu aqueux E₂. On obtient ainsi une émulsion E₁/H/E₂ dans laquelle un soluté du milieu aqueux intérieur E₁ est théoriquement séparé du milieu continu E₂, et ne s'y dissout donc pas. Le cas contraire H₁/E/H₂ est évidemment aussi possible. Pour une revue des émulsions multiples et de leur préparation, on peut se référer à l'un des documents suivants : S. Matsumoto et al., "Formation and Application of Multiple Emulsions", J. Dispersion Science and Technology, 10, 455-482 (1989), ou C. Prybilsky et al., "W/O/W Multiple emulsions: Manufacturing and Formulation considerations", Cosmetics and Toiletries, 106, 143-150 (1994). De nombreux brevets concernent la préparation et surtout la stabilisation d'émulsions multiples, et leurs applications à la cosmétique. On citera, dans le cas des émulsions E/H/E : GB 1541463 (LION Dentifrice Co.), WO 9517155 (Beierdsdorf), WO 9422414 (Henkel), FR 9302795 (Roussel-Uclaf), EP0731685 (IFAC), EP0692957 (Goldschmidt), US 5478561 (Lancaster) et EP 92915365 (Emory Univ.). Ces documents ne représentent qu'un échantillon des nombreux brevets dans le domaine, restreint principalement à la cosmétique et à la pharmacie.

Tous ces documents présentent des procédés classiques de préparation d'émulsions, en partant initialement d'une phase aqueuse émulsionnée à l'aide de divers tensioactifs dans un milieu huile. Cette première émulsion est ensuite elle-même émulsionnée dans un milieu continu aqueux. Les méthodes pour préparer la première émulsion sont des méthodes classiques, qui peuvent être classées en trois méthodes principales : la dispersion mécanique, l'inversion de phase et l'émulsification spontanée. Le document EP 92915365 (Emory Univ.) décrit avec précision ces différentes méthodes, et donne plusieurs références générales. En principe toutes les méthodes utilisent pour la mise en émulsion de l'eau E₁ (phase interne) dans l'huile un tensioactif de basse HLB, typiquement inférieur à 8, en général de nature non-ionique. Au contraire, l'émulsion du système E₁/H dans E₂ utilise un tensioactif de haute HLB, qui peut être non-ionique ou ionique. De nombreux additifs ont été décrits et leur usage breveté pour essayer de stabiliser ces systèmes complexes. La principale difficulté vient de la faible stabilité de l'émulsion E₁/H et de la tendance du tensioactif utilisé pour la seconde émulsion à déstabiliser la première. Parmi les additifs les plus utilisés, on peut citer les sucres (cf. GB 1541463), et les polymères, destinés par exemple à gélifier la phase aqueuse (cf. FR 9302795). De plus on trouve dans la littérature plusieurs exemples d'utilisation de tensioactifs polymères, qui stabilisent visiblement les émulsions multiples (cf. par exemple GB 1541463, qui utilise des Pluronic®, US 5478561 qui utilise des esters de polyglycérol; ou WO 9422414 qui utilise des dérivés de polyakylène).

On trouve dans la littérature de nombreux documents décrivant des milieux dans lesquels un principe actif se trouve encapsulé au sein de vésicules dites vésicules lamellaires comprenant au moins une bicouche de tensioactifs. Ces vésicules sont souvent désignées par vésicules unilamellaires, paucilamellaires ou multilamellaires suivant qu'elles comprennent une, un nombre limité ou un nombre important de bicouches. Les liposomes et les niosomes® constituent des exemples de vésicules lamellaires à base de tensioactifs.

Parmi les vésicules multilamellaires, on distingue celles ci-après désignées par vésicules à structure en oignon, qui sont des vésicules de forme sensiblement sphérique constituées d'une succession régulière de bicouches concentriques et, cela du centre à la périphérie des vésicules.

De telles vésicules se distinguent clairement des liposomes multilamellaires classiques par la régularité de l'empilement des bicouches de tensioactifs qui les constituent. La régularité de cet empilement résulte du caractère thermodynamique des vésicules et de leur symétrie cristal-liquide.

Ces structures peuvent être mises en évidence par examen microscopique des compositions. L'observation se fait en utilisant un microscope optique en lumière polarisée, dans lequel une phase lamellaire, biréfringente, est visible. Elle se manifeste par une texture caractéristique, liée à la présence des défauts (joints de grains) entre les domaines de phase orientés différemment. Dans le cas de la phase concentrée de vésicules, la texture est caractérisée par son caractère uniforme et fin, relié à la taille des vésicules. Dans la phase dispersée de vésicules, celles-ci sont visibles sous la forme de points plus ou moins résolus (en fonction de la taille), légèrement biréfringents. La biréfringence ne s'observe que lorsque la dispersion n'est pas trop diluée ou lorsque les vésicules sont suffisamment grosses (typiquement d'un diamètre supérieur à 5 µm). Il y aura donc lieu, si la dispersion est relativement diluée de procéder à une opération préalable de concentration pour mettre clairement en évidence la biréfringence caractéristique de la présence de ces vésicules.

De telles vésicules peuvent être obtenues par transformation d'une phase cristal-liquide lamellaire incorporant au moins un agent tensioactif sous l'effet d'un cisaillement. Des exemples de préparation et d'utilisation de telles vésicules multilamellaires sont, en particulier, donnés dans les demandes internationales WO 93/19735, WO 95/18601, WO 95/19707, WO 97/00623, WO 98/02144.

Les vésicules multilamellaires de tensioactifs, en particulier les vésicules à structure en oignon, sont des systèmes qui peuvent encapsuler ou incorporer des actifs, en créant un milieu intérieur, différent du milieu extérieur, au sein duquel sont retenus les principes actifs. La rétention de l'actif à l'intérieur de la vésicule a deux origines :
✔ Thermodynamique : la différence d'affinité de l'actif entre le milieu extérieur et le milieu intérieur entraîne son partage entre les deux milieux. De ce fait, dans l'exemple d'une dispersion aqueuse des vésicules, un actif amphiphile sera préférentiellement localisé au sein des vésicules, alors qu'un actif très hydrophile se localisera plutôt dans le milieu extérieur et ne sera donc que très faiblement encapsulé.
✔ Cinétique : chaque membrane à base de tensioactifs forme une barrière de diffusion qui ralentit le passage et donc la fuite vers l'extérieur de l'actif. Ce mécanisme est d'autant plus efficace que l'actif est une grosse molécule, dont le coefficient de diffusion sera faible.

On remarque donc à l'évidence qu'une petite molécule très hydrophile ne sera pas ou très peu encapsulée dans les vésicules, puisque son affinité lui fera préférer le milieu extérieur (toujours dans l'hypothèse d'une dispersion des vésicules dans un milieu aqueux) et que les barrières formées par les bicouches de tensioactifs ne seront que peu efficaces pour la retenir. Par petite ou grosse molécule, on entend une molécule dont la masse molaire est respectivement inférieure ou supérieure à 500 ou 1000 g/mol. Le même raisonnement tient pour l'encapsulation d'une molécule très lipophile, lorsque les vésicules sont dispersées dans un milieu huileux.

De la même manière, et à un degré encore plus marqué, les mêmes mécanismes sont mis en jeu lors de l'encapsulation de molécules dans des liposomes classiques, qui sont des vésicules formées d'un petit nombre de bicouches entourant un (ou plusieurs) coeur aqueux. Dans ce cas, d'une part le milieu du coeur aqueux a une grande similitude avec le milieu extérieur, donc la différence d'affinité de l'actif encapsulé sera très faible, et d'autre part le faible nombre de membranes implique une barrière globale de diffusion beaucoup moins efficace.

Il y a donc une nécessité technique d'améliorer les systèmes d'encapsulation basés sur des membranes de tensioactifs, afin en particulier de leur conférer une meilleure étanchéité. En effet, il y a peu de latitude de réglage possible sur le paramètre thermodynamique, si ce n'est un choix particulier de tensioactifs. Cependant, lorsqu'un produit est soluble dans l'eau, la modification du tensioactif ne peut apporter quasiment aucune amélioration sur son coefficient de partage entre l'eau de la phase extérieure et l'intérieur de la vésicule. De plus le milieu extérieur est souvent un milieu complexe contenant lui-même des tensioactifs (cas des émulsions ou des shampooings) ou d'autres composants (polymères, électrolytes...) susceptibles d'accroître l'affinité de l'actif pour ce milieu, et donc de défavoriser encore son coefficient de partage avec l'extérieur.

Le seul moyen efficace a priori est donc de jouer sur la cinétique de fuite. Pour cela on peut modifier l'étanchéité des barrières, par exemple en changeant de tensioactifs ou renforcer cette étanchéité par incorporation d'un polymère dans les membranes ou dans les couches aqueuses. Cette méthode se heurte à des difficultés d'ordre pratique (les tensioactifs utilisables pour former les membranes ont tous des propriétés de diffusion assez similaires) mais aussi théoriques : l'introduction d'un polymère dans une couche de quelques nanomètres n'apporte, dans bien des cas, qu'une barrière de diffusion relativement peu efficace, la couche de polymère étant quasi monomoléculaire.

Une autre méthode consiste à enrober la vésicule dans une "coquille" de polymère par une méthode classique d'encapsulation par polymère comme la coacervation par exemple. Cette méthode, si elle est séduisante présente quelques difficultés, d'une part sur le plan de sa réalisation, et d'autre part sur les caractéristiques des objets obtenus. Les vésicules faites de membranes de tensioactif ont en général des tailles voisines du micromètre, alors que les capsules obtenues par coacervation ont des diamètres compris entre quelques dizaines et quelques centaines de micromètres. De plus, la coacervation est habituellement mise en oeuvre en utilisant une émulsion, le ou les polymères lors de leur "insolubilisation" venant s'adsorber à l'interface entre l'huile et l'eau autour de chaque gouttelette de l'émulsion. Il n'est pas sûr que l'adsorption du polymère soit possible ou au moins efficace à l'interface entre l'eau et la couche externe de tensioactif des vésicules. Les techniques de coacervation ne sont donc pas parfaitement adaptées à l'enrobage de vésicules à base de tensioactifs. De plus, les objets obtenus par cette technique sont des microcapsules qu'il faut rompre pour libérer le principe actif, au contraire des vésicules qui libèrent leur principe actif lentement par diffusion. Enrober les vésicules par une coque polymère va donc profondément changer la nature des vésicules et surtout leur destination et leur utilisation. Il en va de même des autres techniques d'enrobage par polymère, telles que l'atomisation.

Il a été également décrit dans le brevet US 5 256 422 des milieux constitués d'une émulsion de type eau dans l'huile, dans lesquels les gouttelettes d'eau contiennent des vésicules lipidiques paucilamellaires en dispersion.

Les inventeurs de la présente invention ont maintenant découvert qu'il était possible de réaliser des milieux dispersés ne présentant pas les inconvénients de ceux de l'art antérieur précédemment décrits en utilisant des vésicules multilamellaires à base de tensioactifs comme milieu interne et en les dispersant dans une phase hydrophobe que l'on émulsionne ensuite dans une phase aqueuse.

Les inventeurs ont ensuite pu étendre le même concept aux milieux aqueux obtenus par émulsion dans un milieu hydrophobe d'un milieu aqueux contenant une dispersion de vésicules à base de tensioactifs.

Il est bien connu que les tensioactifs, composés amphiphiles, ont la faculté de s'auto-associer sous forme de membranes qui peuvent soit former des objets refermés sur eux-mêmes, ce sont des vésicules, soit à plus forte concentration s'organiser sous forme de structure lyotrope ayant une organisation cristal-liquide.

Les inventeurs de la présente invention se sont ensuite aperçus que les mêmes avantages pouvaient être obtenus en remplaçant la phase initiale de vésicules par toute phase contenant des membranes de tensioactifs dans un état organisé.

De telles phases contenant des membranes dans un état organisé peuvent en particulier être toute phase de type phase lyotrope susceptible d'être dispersée dans un milieu.

Par phase lyotrope, on entend toute phase organisée ayant une symétrie cristal-liquide faite d'au moins un tensioactif et d'un milieu soit polaire, par exemple aqueux, soit apolaire, par exemple huileux. Les phases lyotropes ne sont pas uniquement lamellaires (encore appelées smectiques A), mais peuvent avoir d'autres structures, de symétries différentes : hexagonale, cubique, etc.

Les phases lyotropes sont des phases condensées de membranes de tensioactifs, en général concentrées en tensioactifs, à l'équilibre thermodynamique, les membranes étant séparées les unes des autres par un milieu de polarité différente. Si le milieu est plus polaire que la membrane de tensioactif (par exemple de l'eau) on a une phase directe, dans le cas contraire (par exemple de l'huile) on a une phase inverse. (On peut trouver une description et des schémas de ces phases dans C.L. Khetrapol, A.C. Kunwar, A.S. Tracy, P. Diels, in Nuclear magnetic resonance studies in lyotropic liquid-crystals, 1975.)

Les phases lamellaires en sont des exemples simples à visualiser, dans lesquelles les membranes, globalement planes, sont simplement empilées les unes sur les autres, et séparées par des couches de milieu polaire ou d'huile. Dans les phases hexagonales, les tensioactifs forment des tubes arrangés dans l'espace selon une disposition hexagonale. Toutes ces phases, sont anisotropes et présentent un ordre cristal-liquide. Il existe aussi des phases cubiques. De plus, ces phases présentent des défauts d'orientation, et sont constituées en général d'une multitude de "grains" séparés par des lignes de défauts (joints de grains) qui sont autant de zones de fragilité permettant de les disperser (à l'instar d'une poudre pour un cristal).

Selon un premier aspect de l'invention, il est maintenant proposé un nouveau milieu complexe constitué d'un premier milieu sous forme de gouttelettes contenant une dispersion d'une phase lyotrope formée de bicouches de tensioactifs, lesdites gouttelettes étant en émulsion au sein d'un deuxième milieu dit phase continue non miscible au premier milieu.

Ce nouveau milieu et sa méthode de préparation se distinguent considérablement des émulsions multiples de l'art antérieur dans la mesure essentiellement où l'on ne part pas, selon la présente invention, d'une émulsion mais d'une dispersion d'objets déjà préformés, ce qui évite notamment de recourir à un cisaillement important tel qu'il est nécessaire dans le cas d'une émulsion multiple pour réaliser la première émulsion.

Un tel milieu se distingue fondamentalement d'une émulsion multiple, du fait que dans le cas de ce milieu la phase interne est formée et structurée préalablement à l'étape de dispersion alors que dans le cas d'une émulsion multiple, on part d'une phase aqueuse que l'on met en émulsion de manière classique dans le milieu huileux. Cette différence a des conséquences non négligeables, du fait que cette préparation préalable confère à la phase interne une bien meilleure stabilité liée à la stabilité intrinsèque de cette phase préexistante.

Un autre avantage est que, dans le cas des vésicules, la taille des objets dispersés est fixée préalablement à l'étape de dispersion.

Un autre avantage dans le cas des milieux de la présente invention est que, du moins dans le cas des milieux lyotropes et des vésicules multilamellaires à structure en oignon, les objets dispersés sont des objets possédant une structure interne découlant d'un équilibre thermodynamique.

Un autre avantage des objets dispersés dans les milieux de la présente invention est qu'ils ne sont pas a priori susceptibles de subir des phénomènes de coalescence et de mûrissement d'Ostwald, causes majeures de déstabilisation des émulsions multiples.

Un autre avantage des milieux de l'invention est qu'ils présentent les caractéristiques organoleptiques (toucher, consistance...) des émulsions multiples et constituent, en conséquence, des systèmes particulièrement intéressants pour l'industrie cosmétique ou dermatologique où ils peuvent être utilisés en tant que véhicules de principes actifs aussi bien hydrophiles que lipophiles et aussi en tant que bases de produits cosmétiques ou dermatologiques à action topique.

Par ailleurs, la présence dans les milieux complexes de l'invention de la phase contenant des bicouches confère au milieu de l'invention des avantages complémentaires. En effet, il est possible d'améliorer la protection et/ou de contrôler le relargage d'un principe actif, en particulier d'un principe actif chimique ou biologique, en incorporant ce principe actif au sein de la phase contenant les bicouches.

Une autre utilisation particulièrement intéressante consiste à utiliser les milieux de l'invention comme de véritables micro-réacteurs, permettant d'isoler temporairement certains réactifs et/ou de contrôler leur pH.

Une autre utilisation des milieux complexes de l'invention est qu'il est possible de les utiliser comme vecteurs d'antigènes, conduisant à une réponse immunitaire amplifiée.

En effet, la vaccination moderne, en particulier celle utilisant des antigènes sous unitaires comme les protéines recombinantes, les glycoprotéines, les peptides ou les polysaccharides, est basée sur l'induction d'une réponse immunitaire par l'administration de l'antigène par différentes voies. Cependant cette réponse est en général insuffisante pour apporter une protection vaccinale lorsque l'antigène est administré directement. L'induction d'une protection efficace nécessite l'utilisation d'adjuvants ou de vecteurs, capables d'amplifier suffisamment la réponse du système immunitaire à l'antigène administré. De nombreux systèmes ont été développés pour répondre à cette exigence. Quelques exemples peuvent être trouvés, par exemple dans Allison A.C., Arch. Immunol Ther Exp., 1997, 45 p 141-7, O'Hagan D.T., J. Pharma. Pharmacol., 1998, 50 p 1-10 ou Bennett B. et al., J. Immunol. Methods, 1992, 153 p 31-40. Plusieurs des exemples cités dans ces articles font apparaître l'efficacité de l'utilisation d'émulsion de l'antigène dans une huile afin de servir d'adjuvant pour cet antigène.

Le brevet européen EP 0480 981 décrit également des émulsions multiples de type E/H/E utilisables comme vaccins.

Les inventeurs ont maintenant observé que les milieux de l'invention constituaient de remarquables vecteurs d'antigènes. Plus précisément, il est apparu que l'on pouvait incorporer un antigène dans une phase contenant des bicouches de tensioactifs dans un état organisé, de préférence dans des vésicules multilamellaires, de préférence dans des vésicules multilamellaires à structure en oignon et qu'il était ensuite possible, par dispersion de cette phase contenant des bicouches dans un état organisé dans une huile puis mise en émulsion de cette huile dans un tampon adéquat, d'obtenir une préparation particulièrement immunogène qui induit une réponse immunitaire beaucoup plus forte que l'antigène seul ou que l'antigène simplement incorporé au sein de ladite phase contenant des bicouches à base de tensioactifs.

Enfin, une autre application consiste à utiliser ces milieux pour préparer des microsphères constituées de polymères renfermant un actif.

Ainsi, selon un deuxième aspect, l'invention couvre diverses applications des milieux complexes de l'invention liées aux avantages énumérés ci-dessus.

D'autres avantages justifiant les applications des milieux de la présente invention apparaîtront plus clairement au vu de la description et des exemples qui suivent.

Selon une de ses caractéristiques essentielles, l'invention concerne un milieu complexe constitué d'un premier milieu sous forme de gouttelettes dans lesquelles est dispersée une phase contenant des bicouches organisées de tensioactifs, lesdites gouttelettes étant en émulsion au sein d'un deuxième milieu dit phase continue non miscible au premier milieu.

Les tailles des gouttelettes du premier milieu sont avantageusement comprises entre 1 et 100 µm, de préférence entre 1 et 50 µm.

L'émulsion contient avantageusement de 1 à 90 % en masse de milieu dispersé dans le milieu continu, de préférence de 1 à 60%.

Les bicouches, sous forme organisée, représentent avantageusement, en masse, de 1 à 90 %, de préférence de 25 à 75 % par rapport aux gouttelettes.

Le premier milieu pourra être un milieu aqueux et la phase continue sera alors un milieu hydrophobe.

Toutefois, selon une variante préférée de l'invention, le premier milieu est un milieu hydrophobe et la phase continue est un milieu aqueux.

A titre de milieu hydrophobe, on utilisera, par exemple, une huile minérale ou végétale, une huile de silicone ou un solvant organique non miscible à l'eau.

Par phase contenant des bicouches organisées, on entend aussi bien des systèmes lyotropes que des vésicules lamellaires.

Toutes les phases lyotropes, dès l'instant où elles sont dispersables dans un milieu pourront être utilisées pour former des dispersions complexes selon l'invention.

Les milieux préférés de l'invention contiennent des vésicules multilamellaires en dispersion au sein d'un milieu lui-même en émulsion dans une phase continue non miscible à ce premier milieu.

Les milieux préférés selon l'invention contiennent une dispersion de vésicules multilamellaires à structure en oignon telles qu'elles ont été définies précédemment.

La taille des vésicules multilamellaires est avantageusement comprise entre 0,1 et 20 µm, de préférence entre 0,1 et 10 µm.

Les proportions de vésicules dans les gouttelettes, avantageusement dans des gouttelettes d'huile, sont de préférence comprises entre 1 et 90 % en masse, typiquement comprises entre 25 et 75 %.

Par mesure de simplification, la description plus détaillée qui va suivre sera faite dans le cas de vésicules à structure en oignon dispersées dans un milieu hydrophobe, lui-même émulsionné dans un milieux aqueux.

Toutefois, l'homme du métier pourra aisément généraliser les informations contenues dans le présent document en les étendant aux systèmes inverses dans lesquels des vésicules sont dispersées dans un milieu aqueux, puis cette dispersion est émulsifiée dans un milieu hydrophobe. L'homme du métier n'éprouvera pas non plus la moindre difficulté à généraliser l'enseignement de ce document au cas de phases organisées de bicouches, qu'il s'agisse de dispersions directes ou inverses.

Ainsi donc, l'enseignement de ce document sera aisément généralisé aux systèmes inverses dans lesquels des objets hydrophobes sont dispersés dans un milieu aqueux et cette dispersion est utilisée dans une émulsion de type eau-dans-huile. L'homme du métier sait, en effet, que des phases lyotropes dans lesquelles des membranes de tensioactifs sont séparées par des couches d'huile existent.

De telles phases peuvent être dispersées en milieu aqueux pour donner une dispersion aqueuse de petits "grains" de phases lyotropes. Ce milieu aqueux peut ensuite être utilisé pour préparer une émulsion de type eau-dans-huile. On obtient ainsi l'équivalent d'une émulsion multiple H/E/H. Des exemples d'émulsions multiples de type H/E/H (encore désignés par émulsion de type O/W/O correspondant à l'abréviation anglaise de Oil/Water/Oil) sont donnés dans les demandes de brevets EP 0 836 847, EP 0 782 646 ou EP 0 559 013 ainsi que dans la demande française FR 96.10140 qui utilise des huiles fluorées.

L'homme du métier sait préparer des vésicules, en particulier des vésicules à structure multilamellaire en oignon en utilisant des formulations qui les rendent dispersables dans un milieu hydrophobe. De telles vésicules sont en particulier décrites dans la demande internationale WO 95/18601 à la page 4, ligne 32 ainsi que dans les exemples 11 et 12 de cette même demande.

Il est ensuite possible d'utiliser l'huile dans laquelle ont été préalablement dispersées les vésicules pour faire une émulsion dans un milieu continu aqueux.

L'homme du métier n'aura aucune difficulté à choisir le tensioactif utilisé pour créer l'émulsion H/E externe, de façon à éviter de déstabiliser l'arrangement multilamellaire des vésicules et de les déstructurer en tenant compte en particulier de ce que la forte concentration en tensioactif dans les vésicules, si celui-ci n'est pas judicieusement choisi est susceptible de déstabiliser l'émulsion extérieure sur des courtes périodes de temps. D'autre part, le choix de l'huile n'est pas innocent, car la dispersabilité des vésicules varie fortement selon que l'on choisit une huile végétale, minérale ou de silicone. En général le choix de l'huile va être déterminé par le type d'application envisagé. Il faudra donc adapter le système tensioactif au type d'huile utilisé.

L'homme du métier comprendra aisément qu'en choisissant correctement le système de tensioactifs utilisés pour créer les vésicules, le tensioactif utilisé pour former l'émulsion extérieure et l'huile de la phase intermédiaire, il est possible de conférer une excellente stabilité, ainsi qu'une bonne capacité à retenir un actif hydrosoluble sans diffusion du milieu interne (vésicules) vers le milieu continu aqueux externe.

Ainsi, selon une variante avantageuse, on utilise des tensioactifs polymères, pour former la vésicule et/ou pour émulsionner l'huile contenant la dispersion de vésicules dans l'eau. Les tensioactifs de type polymère sont choisis de préférence dans une famille parmi laquelle on peut trouver des composés d'HLB différente, de manière à pouvoir utiliser un composé de "basse" HLB pour la préparation des vésicules, qui seront ainsi dispersables dans une huile, et un composé issu de la même famille, mais de "haute" HLB pour la préparation de l'émulsion extérieure H/E. Il est clair pour l'homme du métier que la notion de "basse" et "haute" HLB est difficilement chiffrable de façon très précise. Toutefois, il est connu que les tensioactifs de basse HLB sont plus facilement dispersables dans l'huile. Par basse HLB, on peut entendre HLB inférieure à 8, mais il n'y a pas de limite franche. En particulier les phospholipides n'ont pas de valeur d'HLB bien nette, et sont dispersables en milieu huileux et en milieu aqueux. Cependant, on peut tout à fait envisager d'avoir un tensioactif polymère de basse HLB d'une famille donnée pour la préparation des vésicules et un tensioactif de haute HLB d'une autre famille pour la mise en émulsion aqueuse de la dispersion huileuse de vésicules. Plusieurs familles principales répondent à cette attente :
✔ Les poloxamères, polymères di- ou tribloc d'oxyde d'éthylène et d'oxyde de propylène, le rapport entre les longueurs de chaque bloc fixant l'HLB (le poly oxyde de propylène (encore appelé polypropylène glycol) jouant le rôle de partie lipophile, alors que la partie basée sur l'oxyde d'éthylène est hydrophile). Ces composés sont en particulier représentés dans la famille des PLURONIC® et LUTROL® de BASF,
✔ Les copolymères de polyalkylène glycol et d'alkyl glycol. La longueur de la partie PEG et l'éventuelle méthoxylation des groupes hydroxyl terminaux permet de modifier l'HLB du composé dans une large gamme. Ainsi un copolymère méthoxy PEG-17 dodécyl glycol aura une HLB de 21, et sera donc adapté à l'émulsification de la dispersion huileuse de vésicules dans l'eau, alors qu'un copolymère de PEG-45 et de dodécyl glycol aura une HLB de 4,4 et sera donc adapté à la formulation des vésicules dispersables dans l'huile. Ces composés sont par exemple commercialisés par AKZO NOBEL, dans la gamme ELFACOS®.
✔ Les polyglycérides, esters d'acides gras et de polymères du glycérol. En jouant sur la longueur de chaîne de l'acide gras, sur le nombre de chaînes substituantes et sur le degré de polymérisation du glycérol, on peut faire varier l'HLB dans une large gamme,
✔ Les éthers d'alcools gras et de polymères de glycérol, dans lesquels la fonction ester des polyglycérides a été remplacée par une fonction éther, plus résistante à l'hydrolyse,
✔ Les esters d'acides gras et de polyéthylène glycol, encore appelés polyoxyl n stéarate, dans le cas d'un ester de l'acide stéarique, où n est la longueur moyenne de la chaîne polyéthylène glycol. La chaîne polyéthylène glycol peut être estérifiée à une seule ou aux deux extrémités.
✔ Les éthers d'alcools gras et de polyéthylène glycol, encore appelés poloxyl n alkyl éthers, où n représente le nombre moyen d'unités oxyde d'éthylène dans la chaîne.
✔ Les mélanges d'esters de glycérol et d'esters de polyéthylène glycol. En jouant sur la longueur de chaîne de l'acide gras, sur le nombre de chaînes et sur leur substitution, on peut faire varier la HLB. Ces produits sont commercialisés par exemple par GATTEFOSSE (Lyon, France) sous les noms GELUCIRE® ou LABRAFIL®.
✔ Huile de ricin hydrogénée polyoxyéthylénée (ester de glycérol éthoxylé), résultant de la réaction de l'huile de ricin hydrogénée avec l'oxyde d'éthylène. La HLB est réglée par le nombre d'oxyde d'éthylène.
✔ Huile de ricin polyoxyéthylénée (ricinoléate de glycérol éthoxylé)
✔ Polyméthylcyclosiloxane par exemple, les produits des familles des cyclomethicone et dimethicone copolyol, comme les produits de la société DOW CORNING (DC3225C et DC 5200).

Ces familles sont données à titre d'exemples et ne constituent pas une liste exhaustive. La notion de polymère est prise ici au sens le plus large de molécule de masse molaire importante, certains des composés pouvant être considérés comme des oligomères, d'autres n'étant pas des polymères au sens strict de molécule formée d'une répétition de motifs identiques. Il est difficile de définir un minimum de masse moléculaire pour parler de polymère. On peut se fixer comme limite une masse molaire supérieure à 1 000 Da, qui est une valeur à partir de laquelle la diffusion commence à être assez faible.

L'un au moins des tensioactifs est avantageusement de type polymère, mais il n'est nullement indispensable que des tensioactifs polymères soient utilisés à la fois dans les vésicules et dans l'émulsion extérieure. Bien sûr l'utilisation de tensioactifs polymères dans les deux compartiments renforce la stabilité et surtout l'étanchéité de l'ensemble, mais dans le cas où le soluté à encapsuler à l'intérieur des vésicules n'est pas trop petit, des vésicules formées à partir de tensioactifs non polymères peuvent suffire à obtenir une émulsion stable de gouttelettes d'huile dans l'eau, à l'intérieur desquelles sont dispersées les vésicules encapsulant l'actif. Dans ce cas les vésicules peuvent être obtenues à partir de tout tensioactif dont l'HLB est assez basse pour permettre leur dispersion dans l'huile. C'est le cas des phospholipides ou des sucroesters de basse HLB.

Comme exposé précédemment, les milieux de l'invention peuvent contenir un ou plusieurs principes actifs dans l'un ou l'autre des milieux constituant les milieux complexes de l'invention. Ils peuvent, en particulier, contenir un ou plusieurs principes actifs inclus au sein de la phase contenant les bicouches et, cela, avec les avantages présentés dans la suite de l'exposé et reliés à différentes applications exposées ci-après.

La préparation des milieux de l'invention comprend, d'une façon générale, trois étapes :
- la préparation d'une phase lyotrope formée de tensioactifs,
- la dispersion de cette phase dans un premier milieu,
- la mise en émulsion de la dispersion ainsi obtenue dans un deuxième milieu non miscible au premier milieu, au moyen d'un tensioactif, de préférence un tensioactif de type polymère.

Un ou plusieurs principes actifs peuvent être incorporés aussi bien dans la phase contenant les bicouches, que dans le premier ou le second milieu.

Il pourra s'agir en particulier de principes actifs chimiques ou biologiques.

Dans le cas particulier des milieux préférés de l'invention constitués par des dispersions de vésicules multilamellaires, de préférence de vésicules multilamellaires à structure en oignon dans un milieu hydrophobe que l'on émulsifie dans un milieu continu aqueux, on procédera de préférence selon les trois étapes suivantes :
- Préparation de vésicules à base de tensioactifs, par exemple par la méthode décrite dans l'un des brevets cités précédemment, par cisaillement homogène ou non d'une phase lamellaire lyotrope, mais aussi par toute autre méthode de la littérature conduisant à des vésicules par exemple lipidiques. L'utilisation de vésicules multilamellaires, par leur meilleure stabilité et leur meilleure étanchéité, liée au plus grand nombre de membranes les composant, pourra être préférée dans le cas où ces caractéristiques seraient importantes pour le produit final.
- Dispersion de ces vésicules dans un milieu hydrophobe, par exemple une huile minérale ou végétale ou de silicone, la phase hydrophobe pouvant éventuellement contenir un actif ou un additif lipophile, par exemple un antioxydant ou un conservateur. Un ou des additifs viscosants peuvent aussi être ajoutés à l'huile, si l'on ne veut pas utiliser directement une huile trop visqueuse. L'huile pourra être choisie en fonction de ses caractéristiques de solubilisation de l'actif (on choisira une huile dans laquelle l'actif est le moins soluble pour améliorer l'étanchéité) mais aussi de sa viscosité (une viscosité élevée diminuera la diffusion de l'actif).
- Mise en émulsion de cette dispersion de vésicules dans l'huile dans une phase aqueuse continue, au moyen d'un tensioactif, de préférence un tensioactif polymère. Le milieu extérieur peut être de l'eau, une solution aqueuse ou un milieu lui-même complexe, comme un shampooing ou un gel par exemple.

Les vésicules sont avantageusement formulées avec des tensioactifs de basse HLB, de préférence, qu'ils soient polymères ou non, de manière à ce que leur dispersion dans l'huile soit spontanée ou obtenue par simple agitation. C'est une grande différence avec les méthodes classiques de préparation d'émulsions multiples, dans lesquelles la première étape est une étape d'émulsification, dont l'homme de l'art connaît toutes les difficultés techniques de mise en oeuvre, surtout dans le cas des émulsions inverses. Il est important de noter que l'absence de cette étape initiale d'émulsification d'une phase aqueuse dans l'huile affranchit le procédé de la nécessité parfois rencontrée de soumettre le système à un fort cisaillement ou à des températures relativement élevées, ce qui est très important dans le cas de l'encapsulation de molécules fragiles, par exemple biologiques.

Une autre grande différence avec les systèmes classiques d'émulsions multiples est la présence de la vésicule qui agit comme un réservoir de l'actif en l'encapsulant, ce qui va donner une bien plus grande étanchéité à ce type de système, en limitant les possibilités de migration de l'actif entre la phase aqueuse interne et la phase aqueuse externe continue. De plus le couplage des deux technologies, d'encapsulation par des vésicules de tensioactif et d'émulsions permet d'obtenir une synergie des avantages de ces deux méthodes. De la même manière, un principe actif cosmétique ou thérapeutique d'une préparation topique pourra être protégé par exemple de l'oxydation pendant son stockage (effet de l'émulsion), et mis à disposition lors de l'application sous une forme qui favorise sa pénétration cutanée (effet des vésicules).

Un des principaux intérêts de l'invention est de pouvoir conduire à un système d'encapsulation beaucoup plus étanche que les techniques habituelles utilisant des vésicules à base de tensioactifs, ou que les techniques d'émulsions multiples, sans avoir les inconvénients des techniques d'enrobage par polymères (mise en oeuvre délicate et coûteuse, capsules de taille importante donnant une texture désagréable, nécessité de rompre la coque polymère pour libérer l'actif...).

Selon un autre aspect, l'invention concerne également l'utilisation des milieux de l'invention.

Ces milieux, comme indiqué précédemment, peuvent être en particulier utilisés dans le domaine de la cosmétique ou de la dermatologie. En effet, la cosmétique recherche constamment de nouveaux milieux dont la consistance et la texture soient agréables pour créer de nouvelles bases de produits pour le soin de la peau. Les émulsions multiples, par exemple, sont tout particulièrement recherchées par la cosmétique comme base de crème pour leur toucher particulièrement agréable. Toutefois, elles souffrent de leur manque de stabilité et de leur difficulté de préparation, du fait de la sensibilité de la première émulsion au procédé de fabrication de la seconde émulsion. Les milieux complexes de la présente invention s'avèrent particulièrement intéressants dans cette application car ils présentent les avantages organoleptiques (toucher, consistance...) des émulsions multiples sans en avoir les inconvénients. Ils constituent donc des systèmes particulièrement intéressants pour l'industrie cosmétique, en tant que tel et, cela, indépendamment de leur capacité à incorporer un actif au sein de la phase contenant les bicouches.

D'autres utilisations, quant à elles plus particulièrement reliées aux avantages obtenus du fait de l'incorporation d'un principe actif au sein de la phase contenant les bicouches, sont exposées ci-après.

Ainsi, du fait du contrôle cinétique et non pas thermodynamique de la fuite, l'invention va être particulièrement intéressante dans tous les cas où l'on veut isoler un actif hydrosoluble, mais de faible masse molaire (donc de fort coefficient de diffusion dans les membranes de tensioactifs) d'un milieu continu aqueux. C'est particulièrement utile pour le cas où l'on voudrait protéger ledit actif d'une dégradation, par exemple liée à l'hydrolyse ou à l'oxydation.

Les exemples de tels actifs sont nombreux et on citera à titre d'exemples non exhaustifs :
✔ Les vitamines hydrosolubles
✔ Les hydroxy cétones telles que la dihydroxyacétone et l'érythrulose
✔ Les α-hydroxyacides (acides glycolique, lactique...)
✔ Les électrolytes
✔ Les extraits aqueux ou hydroglycoliques végétaux ou marins
✔ Les oligomères procyanidoliques, et autres dérivés des polyphénols

De plus la méthode pourra être utilisée pour protéger un actif hydrosoluble de petite ou de grande masse molaire, de l'action de petits solutés contenus dans le milieu extérieur, qui, sans cela, migreraient très rapidement du milieu de dispersion vers l'intérieur des vésicules, et pourraient détruire ou modifier l'actif.

Une application particulièrement intéressante est le cas où l'actif doit être maintenu à un pH donné, alors que le produit fini doit, par exemple pour des raisons de sécurité, être formulé à un pH différent. C'est le cas des α-hydroxyacides, qui pour être actifs doivent rester à pH inférieur à 3, mais qui sont introduits dans des préparations cosmétiques dont le pH doit être voisin de 7. C'est le cas aussi du thioglycolate de potassium utilisé dans les crèmes dépilatoires, qui est actif à pH supérieur à 9, mais qui est préférablement présenté dans des crèmes à pH voisin de 7. En créant une barrière cinétique entre milieu extérieur et milieu intérieur, l'invention permet d'atteindre une telle performance.

D'une manière plus générale, l'invention permet de disposer de véritables micro-réacteurs, dans lesquels un réactif est incorporé dans un compartiment du système et l'autre réactif d'une réaction souhaitée est inclus dans l'autre compartiment. La réaction pourra se produire soit par déclenchement, grâce à la rupture de l'émulsion externe (sous l'effet d'une modification de température ou par addition d'un additif adéquat par exemple) soit au contraire très lentement par diffusion des réactifs, celle-ci pouvant être réglée par le choix des tensioactifs et de l'huile intermédiaire.

Les milieux complexes selon l'invention peuvent également être utilisés comme vecteur d'antigène afin d'induire une réponse immunitaire suffisamment forte. A cet effet, l'antigène sera inclus au sein de la phase contenant les bicouches organisées.

Selon une variante particulièrement intéressante, l'antigène sera inclus au sein de vésicules multilamellaires, de préférence à structure en oignon, elles mêmes dispersées dans des gouttelettes d'huile en émulsion dans l'eau. L'inclusion de l'antigène dans un tel milieu permet à la fois de vectoriser l'antigène, de le protéger des agressions extérieures, et en particulier d'une destruction par les enzymes présentes dans l'organisme, et de le mettre à disposition du système immunitaire. Par rapport aux émulsions classiques utilisées comme adjuvant d'antigènes, de type eau dans huile, l'invention se présente sous la forme d'une émulsion huile dans eau, beaucoup plus stable et plus facile à injecter ou à administrer.

Selon une autre variante particulièrement intéressante de l'invention, le premier milieu est un milieu hydrophobe contenant une substance susceptible de se solidifier, d'épaissir, de polymériser ou de précipiter, ou une solution dans un solvant non miscible à l'eau susceptible de s'évaporer d'une telle substance. L'avantage d'un tel milieu est de renforcer l'étanchéité de la phase hydrophobe, ainsi que la solidité du système obtenu après émulsification.

En tant que substance susceptible de se solidifier ou d'épaissir, on citera des cires et des polymères, dont le point de fusion ou de fluidification est tel que l'on peut former le système contenant les bicouches au sein de la phase liquide de la substance, puis en abaissant la température, obtenir un système où le milieu hydrophobe est solidifié ou suffisamment épaissi pour ralentir les phénomènes de diffusion.

On peut aussi dissoudre un monomère dans le milieu hydrophobe, ou un polymère susceptible d'être réticulé, et provoquer après la formation de la dispersion complexe la polymérisation du monomère ou la réticulation du polymère par une méthode chimique, thermique, photochimique ou radiochimique. Dans ce cas, on aura remplacé la gouttelette de dispersion de phase contenant des bicouches par une sphère formée d'une matrice polymère incorporant les grains de cette phase, beaucoup plus stable.

De la même manière, on peut utiliser comme milieu hydrophobe pour disperser la phase contenant des bicouches une solution dans un solvant volatil hydrophobe d'un polymère. Après formation de la dispersion complexe, l'évaporation du solvant conduit à une précipitation du polymère sous la forme de sphères dures emprisonnant les grains de phase contenant des bicouches.

L'invention est donc aussi une méthode originale de préparation de microsphères se présentant comme des matrices de polymère incorporant un actif, dans ce cas sous la forme de (ou inclus dans des) grains de phase contenant des bicouches, et en particulier de phase lyotrope.

Un tel procédé comprend :
- la préparation d'une phase lyotrope renfermant ledit principe actif,
- la dispersion de cette phase dans un milieu hydrophobe dans lequel se trouve dissous un monomère ou un polymère susceptible d'être réticulé ou dans un milieu constitué d'un solvant hydrophobe dans lequel est dissous un polymère,
- la réalisation d'une émulsion sous forme de gouttelettes de ladite dispersion dans un milieu non miscible au milieu hydrophobe ci-dessus,
- la transformation desdites gouttelettes en grains solides, respectivement par polymérisation du monomère ou réticulation du polymère ou précipitation du polymère.
   Une telle variante est tout particulièrement intéressante dans le cas de vésicules multilamellaires, en particulier dans le cas des vésicules multilamellaires à structure en oignon.
   Cette variante, particulièrement intéressante de l'invention, permet la préparation de microsphères de polymères dans lesquelles peuvent être encapsulés des actifs, en particulier des actifs pharmaceutiques comme par exemple les peptides, les protéines (dont les enzymes), ou toute molécule pouvant bénéficier d'une telle encapsulation (effet retard, protection de la molécule ...). Dans ce cas le polymère pourra être avantageusement choisi parmi les polymères résorbables, utilisables en injection parentérale, comme le PlaGa (polylactide glucoside).
   L'invention propose donc, selon cette dernière variante, un moyen d'incorporer dans un milieu une large gamme d'actifs qui peuvent être soit hydrosolubles soit liposolubles. Toutefois, elle s'applique essentiellement aux principes actifs hydrosolubles.
   Les exemples qui suivent illustrent de façon non limitative la présente invention.
   Plus précisément,
- l'exemple 1 donne un exemple de formulation et illustre la préparation d'un milieu complexe selon l'invention constitué d'une émulsion dans un milieu continu aqueux de gouttelettes d'huile minérale dans lesquelles se trouvent dispersées des vésicules à structure en oignon renfermant de la vitamine C,
- l'exemple 2 met en évidence, par miscrocopie optique en lumière polarisée et en lumière directe la structure des milieux complexes de l'invention, en comparaison avec des milieux sous forme d'émulsion double,
- l'exemple 3 relate une étude cinétique de fuite d'un actif et met en évidence l'intérêt des milieux de l'invention pour isoler un actif du milieu extérieur,
- l'exemple 4 met en évidence la différence de stabilité entre une émulsion double et une dispersion complexe selon l'invention.
- l'exemple 5 met en évidence l'utilisation des milieux de l'invention pour réaliser des milieux complexes présentant des différentiels de pH entre différents compartiments,
- l'exemple 6 illustre une utilisation des milieux complexes selon l'invention pour créer un différentiel de pH.
- l'exemple 7 montre l'intérêt des milieux de l'invention dans la préparation d'une émulsion cosmétique contenant un actif particulièrement instable,
- l'exemple 8 illustre l'utilisation des milieux complexes de l'invention pour préparer des microsphères constituées d'un polymère résorbable,
- l'exemple 9 illustre l'utilisation des milieux complexes de l'invention comme vecteur d'antigène permettant d'obtenir une nette amplification de la réponse immunitaire.

Les figures 1 à 8 sont données en référence aux exemples. Plus précisément :
- les figures 1 à 4 correspondant à l'exemple 2 représentent des clichés obtenus en microscopie optique en lumière directe (figure 1 et figure 2) ou en lumière polarisée (figure 3 et figure 4), avec un objectif de 20x et un oculaire de 10x.

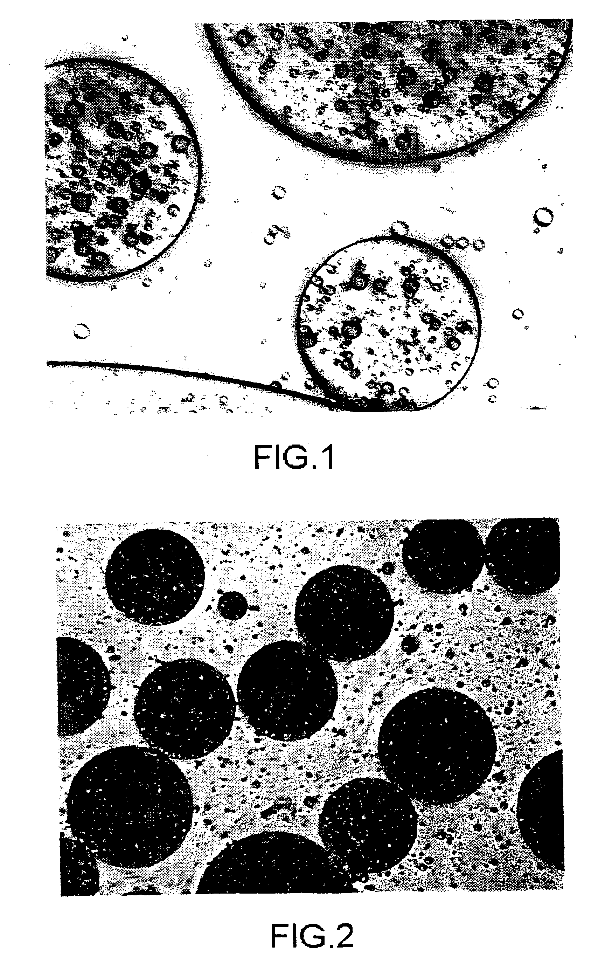

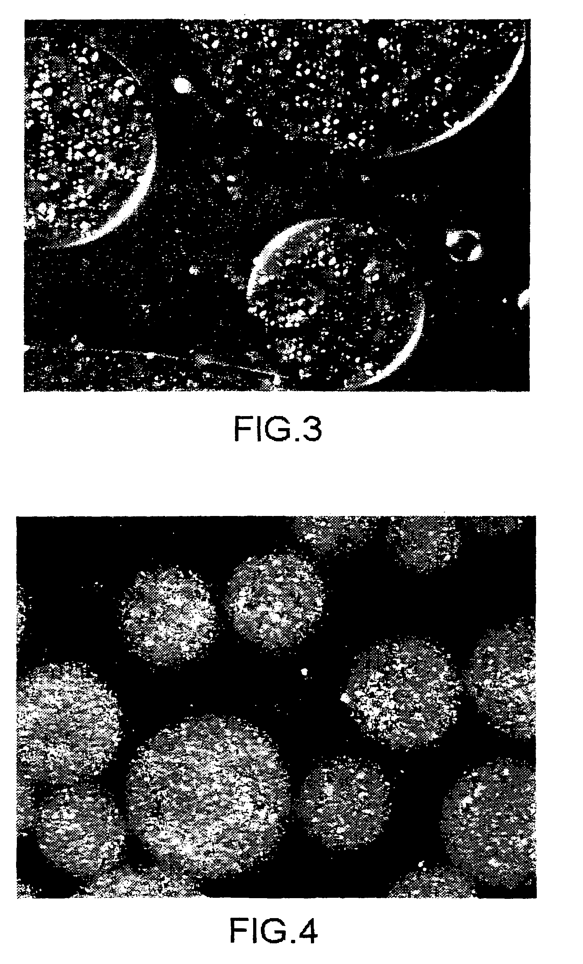

Plus précisément :
. la figure 1 représente un cliché obtenu en microscopie optique en lumière directe dans le cas d'une émulsion double classique,
. la figure 2 représente le cliché obtenu en lumière directe dans le cas du milieu complexe selon l'invention décrit dans l'exemple 2,
. la figure 3 représente le cliché obtenu en lumière polarisée dans le cas d'une émulsion double classique.
. la figure 4 représente le cliché obtenu en lumière polarisée dans le cas du milieu complexe selon l'invention obtenu à l'exemple 2,

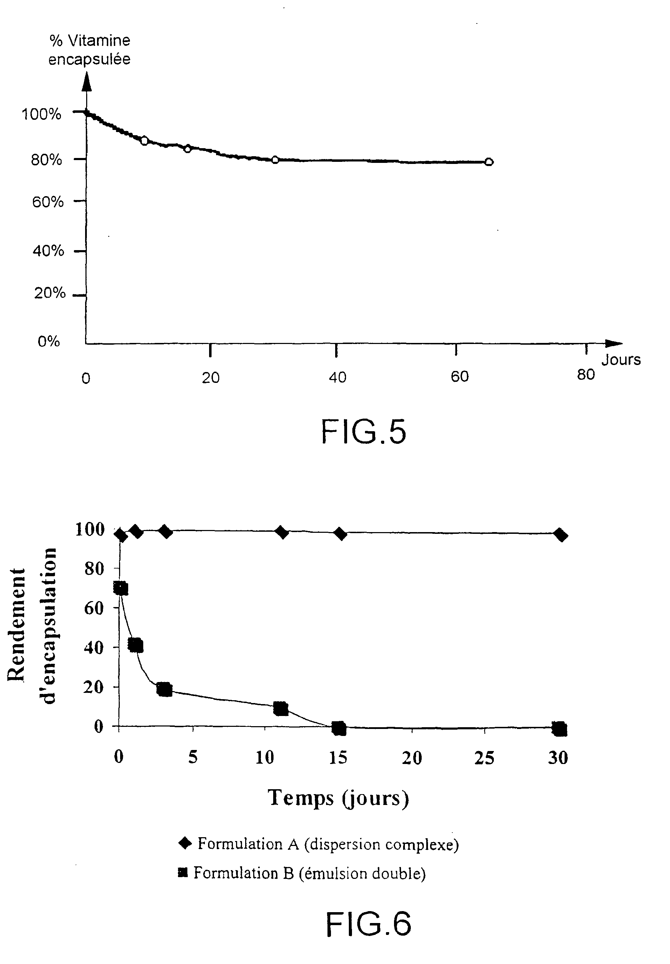

- la figure 5 donnée en réference à l'exemple 3 donne la cinétique de fuite de la vitamine C et représente, plus précisément en ordonnées, le pourcentage de vitamine C qui reste encapsule en fonction du temps indiqué en jours en abscisse,
- la figure 6 donnée en référence à l'exemple 5 donne l'évolution en fonction du temps du rendement d'encapsulation dans les formulations de A et B décrites dans cet exemple

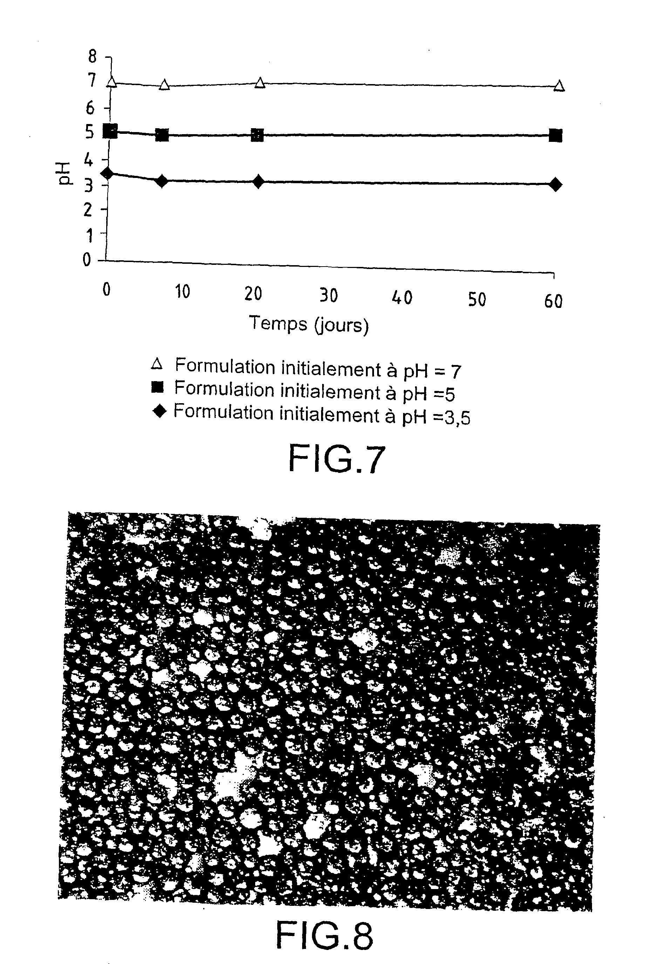

- la figure 7 donnée en référence à l'exemple 6 donne l'évolution en fonction du temps, du pH de trois formulations différentes selon l'invention.
- la figure 8 est un cliché obtenu en microscopie optique en lumière polarisée dans le cas de l'émulsion cosmétique décrite dans l'exemple 7 avec un objectif de 20x et un oculaire de 10x.

### EXEMPLES

### Exemple 1 : Exemple de formulation

La mise en oeuvre du procédé selon l'invention s'effectue en plusieurs étapes. La première étape correspond à la préparation de la phase lyotrope lamellaire. La deuxième étape est celle de dispersion de cette phase dans l'huile et la troisième étape correspond à la mise en émulsion de la dispersion huileuse dans la phase continue aqueuse.

Dans cet exemple, on encapsule une solution aqueuse de vitamine C, qui servira de sonde pour la mesure de fuite de l'exemple 3.

Les pourcentages sont exprimés en poids. Ce mode opératoire est valable pour des quantités de l'ordre de 10 à 100 g.

### Première étape :

### Formulation 1

✔ 40% polyalkylène glycol de type ELFACOS ST9 commercialisé par AKZO NOBEL
✔ 10% polysorbate 60
✔ 30% huile minérale commercialisée en tant qu'huile épaisse par SIGMA
✔ 20% d'une solution aqueuse à 20% de vitamine C.

Après avoir mélangé les trois premiers constituants, à température ambiante, on rajoute la solution à 20% de vitamine C puis on mélange à la spatule pendant 10 à 15 minutes jusqu'à obtention d'un mélange homogène.

### Deuxième étape :

### Formulation 2 :

✔ 20% formulation 1
✔ 80% huile minérale

Les deux constituants sont dispersés à température ambiante puis le mélange est maintenu sous agitation 10 à 15 minutes avec un barreau aimanté.

### Troisième étape :

✔ 20% formulation 2
✔ 80% solution aqueuse de polyalkylène glycol de type ELFACOS OW 100 (AKZO NOBEL) à 1%

Dès que l'on a ajouté la formulation 2, on agite très fortement à la main pendant 10 minutes. On peut aussi utiliser un agitateur magnétique, tournant à grande vitesse.

La préparation est ramenée à pH = 6. La teneur en vitamine C du produit final est de 0,2% (en masse).

On obtient une émulsion, dont l'observation au microscope montre la présence, à l'intérieur des gouttelettes d'huile de vésicules multilamellaires. Leur caractérisation par microscopie optique est réalisée comme dans l'exemple 2.

### Exemple 2 : Visualisation par microscopie optique

Dans cet exemple un produit analogue à celui obtenu dans l'exemple 1, mais dans lequel la vitamine C est remplacée par un oligomère procyanidolique (OPC, référencé "Grape seed extract" obtenu auprès de la société VINYALS, Barcelone Espagne) est comparé avec un produit obtenu selon un procédé d'émulsion double décrit dans la littérature. Le mode opératoire est le même que dans l'exemple 1, mais avec les formulations suivantes.

### 2a - Milieu complexe selon l'invention

### Formulation A

✔ 40% polyalkylène glycol de type ELFACOS ST9 commercialisé par AKZO NOBEL
✔ 10% polysorbate 60
✔ 20% huile minérale
✔ 30% d'une solution aqueuse à 10% d'OPC
✔

### Formulation B

✔ 20% formulation A
✔ 80% huile minérale

### Formulation C

✔ 20% formulation B
✔ 80% solution aqueuse de polyalkylène glycol de type ELFACOS OW 100 (AKZO NOBEL) à 1%

### 2b - Emulsion double (comparative)

L'émulsion double servant à la comparaison est obtenue à partir de la formulation suivante :

Les pourcentages sont exprimés en masse.
A. Une dispersion de tensioactif lipophile dans l'huile est préparée préalablement
   ✔ 1 %polyalkylène glycol de type ELFACOS ST9 commercialisé par AKZO NOBEL
   ✔ 99 % huile minérale
B. Une émulsion eau dans huile est préparée à partir du mélange A
   ✔ 20% de solution aqueuse d'OPC, à 10 %
   ✔ 80% du mélange préparé en A

   Le mélange est effectué à température ambiante, par addition sous agitation magnétique de l'eau dans l'huile.
C. L'émulsion eau dans huile B est mise en émulsion dans un milieu continu aqueux
   ✔ 20 % du mélange obtenu en B
   ✔ 80% solution aqueuse à 1% de polyalkylène glycol de type ELFACOS OW100 (AKZO NOBEL)

Le mélange est obtenu par agitation vigoureuse à la main. On obtient une émulsion blanchâtre.

### 2c - Caractérisation

La méthode de caractérisation est la microscopie optique en lumière polarisée, qui permet de mettre en évidence la biréfringence d'une structure, liée à son caractère anisotrope, et dans le cas de l'invention au caractère cristal-liquide de la structure lamellaire des vésicules.

En se référant aux figures 1 à 4, on constate aisément la différence d'aspect des clichés.

Les figures 1 et 2 montrent qu'en lumière directe les gouttelettes d'émulsion multiple sont claires (transparentes) (voir figure 1) alors que les gouttelettes de la dispersion complexe selon l'invention sont opaques (voir figure 2).

En lumière polarisée l'émulsion multiple (figure 3) ne montre pas de biréfringence (seul un léger effet de bord, classique de l'interface eau/huile est visible), alors que la dispersion complexe de vésicules multilamellaires (figure 4) montre une forte biréfringence à l'intérieur des gouttelettes huileuse. Cette biréfringence démontre que les vésicules ont gardé leur structure lamellaire anisotrope après dispersion dans l'huile. On notera par ailleurs qu'aucune biréfringence n'est observée dans le milieu continu aqueux, ce qui indique que les vésicules sont toutes à l'intérieur des gouttelettes huileuses.

### Exemple 3 : Mesure de la cinétique de fuite de la vitamine C

La mesure de cinétique de fuite consiste à doser la vitamine C dans le milieu continu aqueux de la dispersion complexe préparée dans l'exemple 1. Pour ce faire on sépare tout d'abord les milieux aqueux et huileux de l'émulsion externe, puis on dose par un dosage chimique la vitamine C. Pour vérification, la structure d'un échantillon du système est cassée, par addition de détergent, afin de doser la totalité de la vitamine C (point final).

### a) Séparation

La dispersion complexe de l'exemple 1 est centrifugée à température ambiante, deux fois à 10 000 g. Le surnageant composé de la partie huileuse est séparé, et la fraction aqueuse inférieure est utilisée pour le dosage

### b) Dosage

Le dosage de la vitamine C est réalisée par dosage à l'iode, selon une méthode classique.

20 g de solution aqueuse sont additionnés de 30 g d'eau et de 20 g d'acétone. 4 gouttes d'amidon sont ajoutées pour la visualisation du point de virage. Le dosage est réalisé par addition goutte à goutte d'une solution titrée d'iode.

### c) Point final

Pour la détermination de la quantité totale de vitamine C contenue dans l'échantillon, on casse la dispersion complexe par addition de lauryl sulfate de sodium (SDS). Sur une prise d'essai de 30 g sont ajoutés 3 g de SDS. Après agitation, on obtient une solution homogène, sur laquelle est effectué le dosage à l'iode.

### d) Résultat

La courbe représentée sur la figure 5 donne la fuite de la vitamine C sur une période de 2 mois. On constate qu'elle demeure très faible, inférieure à 20%, et stabilisée bien avant ce temps. On peut donc conclure que la dispersion complexe de l'invention est un moyen efficace pour encapsuler un actif, et l'isoler du milieu extérieur.

### Exemple 4 Comparaison de la stabilité entre dispersion complexe e émulsion double

Afin de comparer la dispersion complexe selon l'invention avec une émulsion double obtenue par une méthode classique, on prépare une émulsion double utilisant les mêmes tensioactifs que ceux entrant dans la formulation de la dispersion complexe. L'amarante, un colorant, est encapsulé dans chaque cas et sert de sonde pour suivre la cinétique de fuite de chacune des formulations.

### Dispersion complexe, formulation A :

La dispersion complexe est préparée selon le mode opératoire donné dans l'exemple 1, en remplaçant la solution aqueuse de vitamine C par une solution aqueuse d'amarante 10⁻² M.

### Emulsion double, formulation B :

L'émulsion double est préparée selon une méthode classique, à partir d'une première émulsion eau dans huile, elle-même émulsionnée dans l'eau. Les tensioactifs employés sont les mêmes que ceux utilisés pour la formulation A.

La première émulsion E₁/H est réalisée de la façon suivante :
Une solution d'amarante 10⁻² M est émulsionnée à 60% dans de l'huile minérale contenant 1% d'un mélange de tensioactifs Elfacos ST-9 / Polysorbate 60 (4/1) à l'aide d'une agitation puissante.

Cette émulsion E₁/H est ensuite émulsionnée dans une phase aqueuse contenant 1 % d'Elfacos OW100.
Le rendement d'encapsulation de l'amarante pour cette formulation B est comparé avec celui de la dispersion complexe, formulation A, au cours du temps, à 22°C. Pour cela, la formulation est séparée par centrifugation, de manière à isoler le milieu extérieur. Sur ce milieu extérieur, une mesure de densité optique par spectrométrie permet de connaître la concentration en amarante qui a fuit. Le résultat apparaît sur la figure 6.

On observe que, alors que dans le cas de l'émulsion double, tout le colorant a fuit en 15 jours, la totalité du colorant est toujours encapsulé dans la dispersion complexe de l'invention, après 30 jours. Cela démontre la différence fondamentale de stabilité, liée à la différence de nature entre une émulsion double et la dispersion complexe selon l'invention.

### Exemple 5 : Différentiel de pH

Une composition selon l'invention est préparée en encapsulant un indicateur de pH (Rouge Congo : bleu violet à pH3, rouge à pH5) à l'intérieur des vésicules multilamellaires.
Formulation des vésicules multilamellaires :
   - 40% polyalkylène glycol de type ELFACOS ST9 commercialisé par AKZO NOBEL
   - 10% Polysorbate 60
   - 30% Huile de paraffine
   - 20% Solution aqueuse d'indicateur coloré, dont le pH est ajusté par de la soude.

Deux échantillons de vésicules sont préparés,
- soit avec une solution d'indicateur coloré à pH 3, les vésicules sont bleues
- soit avec une solution d'indicateur coloré à pH 5,9, les vésicules sont rouges.

Chaque échantillon est alors dispersé à 20% dans de l'huile minérale, les dispersions conservent la coloration et donc le pH des vésicules.

A partir des dispersions huileuses précédentes plusieurs dispersions complexes sont réalisées, en dispersant 20% de dispersions huileuses dans une solution aqueuse de polyalkylène glycol de type ELFACOS OW100 à 1%.

La mise en évidence de l'étanchéité des systèmes vis à vis de l'indicateur coloré sous ses deux formes de pH est réalisée avec les dispersions complexes suivantes :
- Dispersion huileuse de vésicules à pH 3 (bleues) dans une solution d'Elfacos OW100 à pH 3
- Dispersion huileuse de vésicules à pH 5,9 (rouges) dans une solution d'Elfacos OW 100 à pH 6,5.

Des solutions aqueuses d'Elfacos OW100 à 1% d'indicateur coloré sont réalisées à pH 3,3 et 6,5 afin de vérifier que la concentration en indicateur coloré encapsulé dans les vésicules est suffisante pour visualiser la coloration du milieu extérieur en cas de fuite.

Pour les deux systèmes réalisés au pH 3 et 6,5, on n'observe pas de fuite de l'indicateur coloré, des vésicules vers le milieu aqueux extérieur qui reste non coloré.

Ce point étant vérifié, des dispersions complexes avec des différences de pH entre le milieu aqueux encapsulé dans les vésicules et le milieu aqueux extérieur, sont préparées :
- Dispersion huileuse de vésicules multilamellaires à structure en oignon à pH 3 (bleues) dans une solution d'Elfacos OW 100 à pH 6,5
- Dispersion huileuse de vésicules multilamellaires à structure en oignon à pH 5,9 (rouges) dans une solution d'Elfacos OW 100 à pH 3,3.

Dans les deux dispersions complexes, les vésicules conservent leur coloration initiale, liée au pH de préparation, et le milieu extérieur reste non coloré. Ces préparations présentent donc un décalage de pH entre deux compartiments des systèmes, permettant ainsi de conserver l'acidité d'un actif tout en le formulant dans une préparation à un pH plus élevé et vice versa.

### Exemple 6 Stabilité du différentiel de pH

Dans cet exemple, une molécule de petite taille, l'acide salicylique, insoluble dans l'eau est encapsulée à un pH faible (3,5), alors que le pH du milieu extérieur est fixé à différentes valeurs voisines de la neutralité, compatibles avec une utilisation cosmétique (5 et 7)

### Formulation des vésicules multilamellaires:

On prépare une formulation A de vésicules multilamellaires pures selon la composition suivante
❖ 37% polyalkylène glycol de type Eltacos ST9 commercialisé par AKZO NOBEL
❖ 9% Polysorbate 60
❖ 19% Huile minérale
❖ 10% Acide salicylique
❖ 25% Glycérol

On mélange le Polyalkylene glycol (Elfacos ST9), le polysorbate 60, l'huile minérale et l'acide salicylique à l'aide d'un agitateur mécanique en chauffant à 80°C jusqu'à dissolution complete de l'acide salicylique. On rajoute ensuite le glycérol et on poursuit l'agitation jusqu'au retour à température ambiante. On obtient ainsi une phase pure de vésicules multilamellaires, dispersables dans l'huile.

### Dispersion dans l'huile:

La préparation de vésicules multilamellaires concentrées A ainsi obtenue est dispersée dans l'huile minérale dans les proportions suivantes, par agitation mécanique, à température ambiante :
❖ 65% Formulation A
❖ 35% Huile minérale

### Formation de la dispersion complexe :

La dispersion huileuse est alors mise en émulsion en milieu aqueux.

Le milieu aqueux est constitué d'une dispersion dans l'eau de 1% de polyalkylène glycol de type ELFACOS OW100 et de 6% de SEPIGEL 305 (mélange de polyacrylamide, de C₁₃₋₁₄ isoparaffine et de laureth 4 commercialisé par SEPPIC, Paris). Les proportions des deux milieux sont :
❖ 32%Dispersion huileuse
❖ 68% Solution aqueuse

L'émulsification est obtenue par addition lente de la dispersion huileuse sur la solution aqueuse, sous agitation mécanique, à température ambiante.

La dispersion complexe ainsi réalisée a une concentration en acide salicylique de 2% et le pH est de 3,5. Elle a l'aspect et la texture d'une crème cosmétique.

A partir de cette formulation, deux préparations sont réalisées en ajustant le pH avec de la triéthanolamine, à 5 et 7.

La stabilité en pH des trois dispersions complexes est suivie à température ambiante. Le résultat est donné sur le graphe représenté sur la figure 7 où le pH de chacune des préparations est porté en fonction du temps.

On observe que, quelque soit le pH initial, aucune variation de pH n'apparaît dans la crème au cours du temps. Cela signifie que pendant la période observée, aucune fuite d'acide salicylique n'a été constatée. L'huile entourant les vésicules multilamellaires agit dans ce cas comme une barrière évitant toute diffusion entre le milieu interne, où se trouve l'acide salicylique et le milieu externe où le pH a été ajusté.

### Exemple 7 : Préparation d'une émulsion cosmétique

Dans cet exemple est décrit la préparation selon le procédé de l'invention d'une émulsion cosmétique, contenant sous la forme d'une dispersion complexe des vésicules multilamellaires encapsulant un oligomère procyanidolique (OPC). Ce composé est un antiradicalaire puissant, mais particulièrement instable, qui prend une coloration brune sous l'action de l'oxydation.

Les pourcentages sont massiques.

### Etape 1

Cette étape est l'étape de préparation des vésicules multilamellaires encapsulant l'OPC.
✔ 40% Polyalkylène glycol de type ELFACOS ST9 commercialisé par AKZO NOBEL
✔ 10% Polysorbate 60
✔ 20% Huile minérale
✔ 30% Solution aqueuse contenant 10% d'OPC.

Après avoir mélangé ELFACOS, le polysorbate, et l'huile minérale, on ajoute la solution à 10% d'OPC puis on mélange à la spatule pendant 10 à 15 minutes jusqu'à obtention d'un mélange homogène.

On obtient une crème épaisse, phase concentrée de vésicules multilamellaires, contenant 3% d'OPC.

### Etape 2

La formulation obtenue dans l'étape 1, composée d'une phase concentrée de vésicules multilamellaire est dispersée dans une huile minérale.
✔ 20% formulation 1
✔ 80% Huile minérale.

La dispersion s'effectue simplement à température ambiante, par incorporation lente de l'huile minérale sur la crème sous agitation manuelle. La dispersion est ensuite mélangée 10 à 15 minutes avec un barreau aimanté et un agitateur magnétique.

### Etape 3

La dispersion huileuse de vésicules obtenue à l'étape 2 est mise en émulsion en milieu aqueux. Afin d'obtenir la texture et la stabilité souhaitées, on utilise un gélifiant de la phase continue aqueuse.

On prépare une dispersion de polyalkylène glycol de type ELFACOS OW 100 à 1% contenant 1% de SEPIGEL 305 (Mélange de polyacrylamide, de C13-14 isoparaffine et de laureth 4, commercialisé par la société SEPPIC). Cette dispersion est obtenue en incorporant le SEPIGEL à la dispersion du tensioactif, sous forte agitation mécanique.
Formulation
✔ 20% Formulation obtenue à l'étape 2
✔ 80% dispersion de polyalkylène glycol de type ELFACOS OW 100 à 1% contenant 1% de SEPIGEL

La formulation obtenue à l'étape 2 est additionnée à la dispersion de tensioactifs et de polymère sous agitation mécanique.

On obtient une crème blanche, de toucher onctueux, stable plusieurs mois à température ambiante, ne montrant aucune tendance à la décantation ou au crémage.

Comme cela apparaît sur la figure 8, l'observation en microscopie optique en lumière polarisée permet de mettre en évidence la présence des vésicules multilamellaires à l'intérieur des gouttelettes d'huile.

### Exemple 8 : Préparation de microsphères de polymère résorbable

Dans cet exemple, l'invention est utilisée pour préparer des microsphères d'un polymère biodégradable le poly-DL-lactide (Pla) de masse 43000g/mol distribué par la société PHUSYS.

### Formulation :

Une phase concentrée de vésicules multilamellaires est préparée par mélange à température ambiante des constituants suivants :
✔ 40 % Lécithine de soja
✔ 10 % Oléate de sorbitan
✔ 50 % eau.

Cette phase concentrée est dispersée rapidement dans une solution à 10% (en masse) de polymère dans du dichlorométhane. Cette dispersion est réalisée en environ 1 minute, de façon à éviter une dégradation des vésicules. Le rapport entre la masse de polymère en solution et la masse de vésicules dispersée est de 2.

Puis cette dispersion organique de vésicules est mise en émulsion à 3 % dans une solution aqueuse contenant 1% en masse de poly (vinyl alcool) de masse 30000-70000 distribué par la société SIGMA. A l'aide d'un barreau magnétique, on agite la solution tout en procédant à l'addition. Cette agitation est prolongée durant 2 à 3 heures sans boucher le flacon de façon à évaporer le solvant organique.

Une fois terminée l'évaporation on peut observer en microscopie optique des objets sphériques qui ne sont pas détruits par l'ajout de détergents comme le Triton X100 (SIGMA) ou par addition d'une solution contenant 10% de sel de déoxycholate (principal constituant des sels biliaires). Ces détergents sont connus pour solubiliser les structures à base de lécithine. La résistance à ces détergents des objets obtenus dans cet exemple indique que la composition externe de ces vésicules n'est pas le phospholipide.

### Observation en microscopie électronique :

Des clichés de microscopie électronique en transmission montrent à l'intérieur des microsphères de polymère la présence d'une masse sombre dont on ne peut déterminer la composition, alors que des microsphères vides du même polymère (préparées à partir d'une émulsion ne contenant pas les vésicules multilamellaires) montrent un coeur qui est transparent par cette même technique d'observation.

### Encapsulation d'un colorant:

On peut incorporer dans la phase lamellaire initiale, avant l'étape de dispersion dans la solution organique, un colorant hydrophile (bleu de méthylène). On procède ensuite à la préparation des microsphères de polymère Pla selon le même mode opératoire que précédemment. On obtient alors des microsphères de polymère, incorporant des vésicules multilamellaires, encapsulant le colorant.

La mesure du rendement d'encapsulation est réalisée en dosant par spectrophotométrie UV/visible le colorant dans le surnageant, après séparation des microsphères par centrifugation. On trouve un rendement d'encapsulation de 85% ce qui montre qu'on n'a pas dégradé les vésicules lors de l'étape de formation des microsphères de polymère.

### Exemple 9 : Encapsulation d'un antigène pour amplification de la réponse immunitaire

L'invention peut être utilisée pour encapsuler un antigène afin de le vectoriser et ainsi d'amplifier la réponse immunitaire. On démontre ici que l'utilisation d'une dispersion complexe formée à partir de vésicules multilamellaires encapsulant l'antigène permet d'amplifier considérablement la réponse immunitaire.

Le modèle choisi est l'albumine de sérum humain (HSA) injectée par voie sous-cutanée à des souris. La réponse immunitaire est mesurée par test ELISA dosant les anticorps anti-HSA totaux dans le sérum des souris.

### Formulation

Les vésicules multilamellaires sont préparées à partir de :

| | |
|---|---|
| ✔ Oléate de potassium | 5% |
| ✔ Cholestérol | 5 % |
| ✔ Sulfate de cholestérol | 2,5% |
| ✔ Alcool laurique éthoxylé à 4 oxyde d'éthylène (laureth 4) | 2% |
| ✔ PBS 1x | 20% |
| ✔ HSA en solution à 20mg/ml dans PBS 1x | 20% |
| ✔ Phosphatidylcholine à 90% (PC90 Natterman) | 45,5% |

Les cinq premiers constituants sont mélangés puis mis à incuber lh à 90°C, jusqu'à disparition complète des cristaux de cholestérol et de sulfate de cholestérol. Après refroidissement à température ambiante, sont lentement incorporés la solution d'HSA puis la phosphatidylcholine. Le mélange est incubé 2 heures à 37°C, puis cisaillé manuellement pendant 5 minutes.

La pâte concentrée obtenue est séparée en deux lots avant la dispersion. Elle est titrée à 4 mg/g en HSA.

Un lot est dispersé à 5% par addition d'un tampon PBS aqueux. La concentration finale en HSA est de 20 µg pour 100 µl (0,02% en masse).

L'autre lot est dispersé dans une huile minérale, à raison de 200 mg de pâte pour 0,8 ml d'huile. La dispersion est obtenue en additionnant lentement à température ambiante l'huile minérale sur la pâte, sous agitation manuelle.

Cette dispersion huileuse est mise en émulsion comme décrit dans l'exemple 1, par addition de la dispersion huileuse dans une solution aqueuse de polyalkylène glycol de type ELFACOS OW 100 (AKZO NOBEL) à 1%, à raison de 0,25 ml d'huile pour 0,75 ml de solution aqueuse. Le mélange est homogénéisé par une vigoureuse agitation manuelle. Le titre final en HSA est de 20 µg pour 100 µl de dispersion.

### Protocole

Les préparations sont injectées par voie sous-cutanée à des groupes de 4 souris femelles BALB/c, à raison d'une injection à t = 0, puis une injection à t = 10 jours. Un groupe témoin n'est pas injecté, un groupe reçoit la dispersion aqueuse, l'autre groupe reçoit la dispersion complexe. Les souris sont sacrifiées à t = 23 jours, le sang est prélevé dans des tubes héparinés et le sérum isolé par centrifugation.

Le dosage des immunoglobulines totales est réalisé par test ELISA sur ces sérums selon un protocole classique, en utilisant des immunoglobulines de lapin anti immunoglobulines de souris, marquées à la peroxydase. Les immunoglobulines fixées sont révélées par l'addition du substrat de l'enzyme qui donne un produit coloré absorbant à 490 nm. Pour chaque sérum la réaction est effectuée sur une série de 12 dilutions de 1/25 à 1/(25x2¹¹).

### Résultats

La mesure est effectuée par l'enregistrement de la densité optique (DO), en fonction de la dilution du sérum, à 490 nm. Le seuil de positivité est défini comme la valeur moyenne de la DO obtenue sur la première dilution (1/25) avec les sérums des quatre souris témoins non injectées, augmentée de t fois l'écart type sur cette valeur moyenne, ou t est la valeur défini dans la méthode de Student (cf. Pharmacopée Européenne, méthodes statistiques).

Les résultats sont exprimés en titre moyen défini comme le facteur de dilution du sérum pour lequel la DO moyenne sur les quatre souris du groupe est supérieure au seuil de positivité. On obtient les titres suivants :

| | Titre moyen en IgG totales |
|---|---|
| HSA dans PBS | 0 |
| Dispersion aqueuse | 2870 |
| Dispersion complexe | 17900 |

On constate donc une réponse importante en dispersion aqueuse, mais très nettement supérieure par injection de la formulation en dispersion complexe selon l'invention.

## Revendications

1. Milieu complexe constitué d'un premier milieu sous forme de gouttelettes contenant une dispersion d'une phase lyotrope formée de bicouches de tensioactifs, lesdites gouttelettes étant en émulsion au sein d'un deuxième milieu dit phase continue non miscible au premier milieu.

2. Milieu complexe selon la revendication 1, **caractérisé en ce que** la taille des gouttelettes du premier milieu est comprise entre 1 et 100 µm, de préférence entre 1 et 50 µm.

3. Milieu complexe selon l'une des revendications 1 ou 2, **caractérisé en ce que** ladite émulsion contient de 1 à 90 % en masse de milieu dispersé dans le milieu continu.

4. Milieu complexe selon l'une des revendications 1 à 3, **caractérisé en ce que** lesdites gouttelettes contiennent de 1 à 90 % en masse de phase lyotrope formée de bicouches de tensioactifs.

5. Milieu complexe selon l'une des revendications 1 à 4, **caractérisé en ce que** ledit premier milieu est un milieu hydrophobe et ladite phase continue un milieu aqueux.

6. Milieu complexe selon l'une des revendications 1 à 4, **caractérisé en ce que** ladite phase dispersion de phase lyotrope est sous forme d'une dispersion de vésicules multilamellaires, dites vésicules à structure en oignon, de forme sensiblement sphérique, constituées d'une succession régulière de bicouches concentriques et, cela, du centre à la périphérie desdites vésicules.

7. Milieu complexe selon la revendication 6, **caractérisé en ce que** lesdites vésicules multilamellaires à structure en oignon, présentent des dimensions comprises entre 0,1 et 20 µm, de préférence entre 0,1 et 10 µm.

8. Milieu complexe selon l'une des revendications 1 à 7, **caractérisé en ce qu'**il est constitué d'une dispersion de vésicules multilamellaires dites vésicules à structure en oignon, de forme sensiblement sphérique, constituées d'une succession régulière de bicouches concentriques et, cela, du centre à la périphérie desdites vésicules, au sein de gouttelettes d'un milieu hydrophobe, lesdites gouttelettes étant en émulsion au sein d'un milieu aqueux.

9. Milieu complexe selon l'une des revendications 1 à 8, **caractérisé en ce que** la phase continue renferme au moins un agent tensioactif permettant la mise en émulsion desdites gouttelettes.

10. Milieu complexe selon la revendication 9, **caractérisé en ce que** l'un au moins des tensioactifs permettant la mise en émulsion ou entrant dans la composition des bicouches est un tensioactif présentant une masse molaire supérieure à 1 000 Da, de préférence un tensioactif de type polymère.

11. Milieu complexe selon la revendication 10, **caractérisé en ce que** ledit tensioactif de type polymère est choisi parmi les poloxamères, les copolymères de polyalkylène glycol et d'alkyl glycol , les polyglycérides, les éthers d'alcools gras et de polymères de glycérol , les esters d'acides gras et de polyéthylène glycol, les éthers d'alcools gras et de polyéthylène glycol.

12. Milieu complexe selon l'une des revendications 1 à 11, **caractérisé en ce que** ledit premier milieu est un milieu hydrophobe contenant une substance susceptible de se solidifier, d'épaissir, de polymériser ou de précipiter ou une solution d'une telle substance dans un solvant non miscible à l'eau susceptible de s'évaporer.

13. Milieu complexe selon l'une des revendications 1 à 12, **caractérisé en ce qu'**il contient au moins un principe actif.

14. Milieu complexe selon l'une des revendications 1 à 13, **caractérisé en ce qu'**il contient au moins un principe actif chimique ou biologique incorporé au sein de ladite phase lyotrope formée de bicouches utilisée pour la préparation de ladite dispersion.

15. Milieu complexe selon l'une des revendications 1 à 14, **caractérisé en ce que** au moins un antigène est incorporé au sein de ladite phase lyotrope.

16. Procédé pour la préparation d'un milieu selon l'une des revendications 1 à 15, **caractérisé en ce qu'**il comprend :
- la préparation d'une phase lyotrope formée de bicouches de tensioactifs,
- la dispersion de cette phase dans un premier milieu,
- la mise en émulsion de la dispersion ainsi obtenue dans un deuxième milieu non miscible au premier milieu, au moyen d'un tensioactif, de préférence un tensioactif de type polymère, un ou plusieurs principes actifs pouvant être incorporés dans la phase lyotrope et/ou dans le premier milieu et/ou dans le deuxième milieu.

17. Procédé selon la revendication 16, **caractérisé en ce que** ladite phase lyotrope est dispersée sous forme de vésicules multilamellaires dites vésicules à structure en oignon, de forme sensiblement sphérique, constituées d'une succession régulière de bicouches concentriques et, cela, du centre à la périphérie desdites vésicules.

18. Procédé pour préparer des microsphères de polymère incorporant un principe actif, **caractérisé en ce qu'**il comprend :
- la préparation d'une phase lyotrope renfermant ledit principe actif,
- la dispersion de cette phase dans un milieu hydrophobe dans lequel se trouve dissous un monomère ou un polymère susceptible d'être réticulé ou dans un milieu constitué d'un solvant hydrophobe dans lequel est dissous un polymère,
- la réalisation d'une émulsion sous forme de gouttelettes de ladite dispersion dans un milieu non miscible au milieu hydrophobe ci-dessus,
- la transformation desdites gouttelettes en grains solides, respectivement par polymérisation du monomère ou réticulation du polymère ou précipitation du polymère.

19. Microsphères de polymère susceptibles d'être obtenues par le procédé selon la revendication 18.

20. Procédé pour contrôler la libération d'un actif et/ou le protéger d'une dégradation, **caractérisé en ce qu'**il consiste à incorporer ledit actif dans une phase lyotrope formée de bicouches de tensioactifs, à disperser ladite phase dans un premier milieu et à émulsionner ledit premier milieu dans un deuxième milieu non miscible avec ledit premier milieu.

21. Utilisation d'un milieu complexe selon l'une des revendications 1 à 14 pour réaliser un milieu présentant un différentiel de pH.

22. Utilisation d'un milieu complexe selon l'une des revendications 1 à 14 comme base d'une composition topique à usage cosmétique ou dermatologique.

23. Utilisation d'un milieu complexe selon la revendication 15 pour la préparation d'une composition destinée à augmenter la réponse immunitaire vis à vis dudit antigène.

24. Utilisation selon la revendication 23, **caractérisée en ce que** lesdites bicouches sont sous forme de vésicules multilamellaires dites vésicules à structure en oignon, de forme sensiblement sphérique, constituées d'une succession régulière de bicouches concentriques et, cela, du centre à la périphérie desdites vésicules.

## Claims

1. A complex medium constituted of a first medium in the form of droplets containing a dispersion of a lyotropic phase of surfactant bilayers, said droplets being in emulsion in a second medium named "continuous phase", which is immiscible with the first medium.

2. The complex medium according to claim 1, **characterized in that** the size of the droplets of the first medium is between 1 µm and 100 µm, preferably between 1 µm and 50 µm.

3. The complex medium according to claim 1 or 2, **characterized in that** said emulsion contains from 1% to 90% by weight of a dispersed medium in the continuous medium.

4. The complex medium according to one of claims 1 to 3, **characterized in that** said droplets contain from 1% to 90% by weight of a lyotropic phase of surfactant bilayers.

5. The complex medium according to one of claims 1 to 4, **characterized in that** said first medium is a hydrophobic medium and said continuous phase is an aqueous medium.

6. The complex medium according to one of claims 1 to 4, **characterized in that** said dispersion phase of a lyotropic phase is in the form of a dispersion of multilamellar vesicles, so-called "onion" vesicles, of substancially spherical shape, constituted of a regular succession of concentric bilayers from the center to the periphery of said vesicles.

7. The complex medium according to claim 6, **characterized in that** said multilamellar "onion" vesicles are between 0.1 µm and 20 µm in size, preferably between 0.1 µm and 10 µm in size.

8. The complex medium according to one of claims 1 to 7, **characterized in that** said complex medium is constituted of a dispersion of multilamellar "onion" vesicles, of substantially spherical shape, constituted of a regular succession of concentric bilayers from the center to the periphery of said vesicles, within droplets of a hydrophobic medium, said droplets being in emulsion in an aqueous medium.

9. The complex medium according to one of the claims 1 to 8, **characterized in that** the continuous phase contains at least one surfactant enabling said droplets to be placed in emulsion.

10. The complex medium according to claim 9, **characterized in that** at least one of the surfactants enabling the placing in emulsion or entering into the composition of bilayers is a surfactant, with a molar weight greater than 1,000 Da, preferably a polymeric surfactant.

11. The complex medium according to claim 10, **characterized in that** said polymeric surfactant is selected among poloxamers, copolymers of polyalkylene glycol and alkyl glycol, polyglycerides, ethers of fatty alcohols and glycerol polymers, esters of fatty acids and polyethylene glycols, and ethers of fatty alcohols and polyethylene glycol.

12. The complex medium according to one of claims 1 to 11, **characterized in that** said first medium is a hydrophobic medium containing a substance capable to solidify, thicken, polymerize or precipitate, or a solution of one such substance in a solvent that is immiscible with water and capable to evaporate.

13. The complex medium according to one of claims 1 to 12, **characterized in that** said complex medium comprises at least one active ingredient.

14. The complex medium according to one of claims 1 to 13, **characterized in that** said complex medium contains at least one chemical or biological active ingredient incorporated in said lyotropic phase of bilayers used to prepare said dispersion.

15. The complex medium according to one of claims 1 to 14, **characterized in that** at least one antigen is incorporated within said lyotropic phase.

16. A method for the preparation of a medium according to one of claims 1 to 15, **characterized in that** said method comprises:
- preparation of a lyotropic phase of surfactant bilayers,
- dispersion of said phase in a first medium,
- setting in emulsion of the dispersion thus obtained in a second medium immiscible with the first medium, by means of a surfactant, preferably a polymeric surfactant,
one or more active ingredients being able to be incorporated in the lyotropic phase and/or the first medium and/or the second medium.

17. The method according to claim 16, **characterized in that** said lyotropic phase is dispersed in the form of multilamellar "onion" vesicles, of substancially spherical shape, constituted of a regular succession of concentric bilayers from the center to the periphery of said vesicles.

18. A method for the preparation of polymer microspheres incorporating an active ingredient, wherein said method comprises:
- preparation of a lyotropic phase containing said active ingredient,
- dispersion of said phase in a hydrophobic medium in which is dissolved a monomer or a polymer likely to be cross-linked, or in a medium constituted of a hydrophobic solvent in which a polymer is dissolved,
- production of an emulsion in the form of droplets of said dispersion in a medium immiscible with the above-mentioned hydrophobic medium,
- transformation of said droplets into solid particles, by the polymerization of the aforesaid monomer or by the cross-linking and then the precipitation of the aforesaid polymer.

19. Polymer microspheres obtainable by the method according to claim 18.

20. A method for controlling the release of, and/or preventing the degradation of, an active ingredient, **characterized in that** it consists in incorporating said active ingredient in a lyotropic phase of surfactant bilayers, dispersing said phase in a first medium and emulsifying said first medium in a second medium immiscible with said first medium.

21. Use of a complex medium according to one of claims 1 to 14 to produce a medium exhibiting a pH differential.

22. Use of a complex medium according to one of claims 1 to 14 as the base for a topical composition for cosmetic or dermatological use.

23. Use of a complex medium according to claim 15 for the preparation of a composition for increasing the immune response to a given antigen.

24. Use of a complex medium according to claim 23, **characterized in that** said bilayers are in the form of multilamellar "onion" vesicles, of substantially spherical shape, consisting of a regular succession of concentric bilayers from the center to the periphery of said vesicles.

## Patentansprüche

1. Komplexes Medium, bestehend aus einem ersten Medium in Form von Tröpfchen, die eine Dispersion einer lyotropen Phase, gebildet aus Tensiddoppelschichten, enthält, wobei die Tröpfchen in Emulsion in einer zweiten sogenannten kontinuierlichen Phase sind, die mit dem ersten Medium nicht mischbar ist.

2. Komplexes Medium nach Anspruch 1, **dadurch gekennzeichnet, daß** die Größe der Tröpfchen des ersten Mediums zwischen 1 und 100 µm, vorzugsweise zwischen 1 und 50 µm liegt.

3. Komplexes Medium nach einem der Ansprüche 1 oder 2, **dadurch gekennzeichnet, daß** die Emulsion 1 bis 90 Masse-% in dem kontinuierlichen Medium dispergiertes Medium enthält.

4. Komplexes Medium nach einem der Ansprüche 1 bis 3, **dadurch gekennzeichnet, daß** die Tröpfchen 1 bis 90 Masse-% lyotrope Phase, gebildet aus Tensiddoppelschichten, enthält.

5. Komplexes Medium nach einem der Ansprüche 1 bis 4, **dadurch gekennzeichnet, daß** das erste Medium ein hydrophobes Medium ist und daß die kontinuierliche Phase ein wäßriges Medium ist.

6. Komplexes Medium nach einem der Ansprüche 1 bis 4, **dadurch gekennzeichnet, daß** die Dispersionsphase einer lyotropen Phase in Form einer Dispersion von multilamellaren Vesikeln vorliegt, genannt Vesikel mit Zwiebelstruktur, im wesentlichen in Kugelform, bestehend aus einer regelmäßigen Abfolge von konzentrischen Doppelschichten und dies vom Zentrum zur Peripherie der Vesikel hin.

7. Komplexes Medium nach Anspruch 6, **dadurch gekennzeichnet, daß** die multilamellaren Vesikel mit Zwiebelstruktur Abmessungen zwischen 0,1 und 20 µm, vorzugsweise zwischen 0,1 und 10 µm, aufweisen.

8. Komplexes Medium nach einem der Ansprüche 1 bis 7, **dadurch gekennzeichnet, daß** es aus einer Dispersion von multilamellaren Vesikeln besteht, genannt Vesikel mit Zwiebelstruktur, im wesentlichen in Kugelform, bestehend aus einer regelmäßigen Abfolge von konzentrischen Doppelschichten und dies vom Zentrum zur Peripherie der Vesikel hin, in Tröpfchen eines hydrophoben Mediums, wobei die Tröpfchen in Emulsion in einem wäßrigen Medium sind.

9. Komplexes Medium nach einem der Ansprüche 1 bis 8, **dadurch gekennzeichnet, daß** die kontinuierliche Phase wenigstens ein Tensidmittel umfaßt, das das Emulgieren der Tröpfchen erlaubt.

10. Komplexes Medium nach Anspruch 9, **dadurch gekennzeichnet, daß** wenigstens eines der Tenside, die das Emulgieren erlauben oder in die Zusammensetzung der Doppelschichten eintreten, ein Tensid ist, das eine Molmasse über 1000 Da hat, vorzugsweise ein Tensid vom Polymertyp.

11. Komplexes Medium nach Anspruch 10, **dadurch gekennzeichnet, daß** das Tensid vom Polymertyp gewählt ist unter Polyoxameren, Copolymeren von Polyalkylenglycol und Alkylglycol, Polyglyceriden, Ethern von Fettalkoholen und Glycerinpolymeren, Estern von Fettsäuren und von Polyethylenglycol, Ethern von Fettalkohlen und von Polyethylenglycol.

12. Komplexes Medium nach einem Ansprüche 1 bis 11, **dadurch gekennzeichnet, daß** das erste Medium ein hydrophobes Medium ist, das eine Substanz enthält, die in der Lage ist, sich zu verfestigen, zu verdicken, zu polymerisieren oder auszufallen oder eine Lösung einer solchen Substanz in einem Lösungsmittel, das nicht mischbar ist mit Wasser und in der Lage ist, zu verdampfen.

13. Komplexes Medium nach einem der Ansprüche 1 bis 12, **dadurch gekennzeichnet, daß** es wenigstens einen Wirkstoff enthält.

14. Komplexes Medium nach einem der Ansprüche 1 bis 13, **dadurch gekennzeichnet, daß** es wenigstens einen chemischen oder biologischen Wirkstoff, einverleibt in die lyotrope Phase, enthält, die gebildet ist aus Doppelschichten und verwendet wird zur Herstellung der Dispersion.

15. Komplexes Medium nach einem der Ansprüche 1 bis 14, **dadurch gekennzeichnet, daß** wenigstens ein Antigen in die lyotrope Phase einverleibt ist.

16. Verfahren zur Herstellung eines Mediums nach einem der Ansprüche 1 bis 15, **dadurch gekennzeichnet, daß** es umfaßt:
- die Herstellung einer lyotropen Phase, die gebildet ist aus Tensiddoppelschichten,
- die Dispersion dieser Phase in einem ersten Medium,
- das Emulgieren der so erhaltenen Dispersion in einem zweiten Medium, das mit dem ersten Medium nicht mischbar ist, mittels eines Tensids, vorzugsweise eines Tensids vom Polymertyp,
wobei einer oder mehrere der Wirkstoffe in die lyotrope Phase und/oder in das erste Medium und/oder in das zweite Medium einverleibt werden können.

17. Verfahren nach Anspruch 16, **dadurch gekennzeichnet, daß** die lyotrope Phase dispergiert ist in Form von multilamellaren Vesikeln, sogenannten Vesikeln mit Zwiebelstruktur, im wesentlichen in Kugelform, bestehend aus einer regelmäßigen Abfolge konzentrischer Doppelschichten und dies vom Zentrum zur Peripherie der Vesikel hin.

18. Verfahren zur Herstellung von Polymermikrokugeln, die einen Wirkstoff einverleiben, **dadurch gekennzeichnet, daß** es umfaßt:
- die Herstellung einer lyotropen Phase, die den Wirkstoff einschließt,
- die Dispersion dieser Phase in einem hydrophoben Medium, in welchem sich ein Monomer oder ein Polymer gelöst befindet, das geeignet ist, vernetzt zu werden oder in einem Medium, das aus einem hydrophoben Lösungsmittel besteht, in welchem ein Polymer gelöst wird,
- das Ausführen einer Emulsion in Form von Tröpfchen dieser Dispersion in einem Medium, das nicht mischbar ist mit dem obigen hydrophoben Medium,
- die Umwandlung der Tröpfchen zu festen Körnern, jeweils durch Polymerisation des Monomers oder Vernetzung des Polymers oder Fällen des Polymers.

19. Polymermikrokugeln, die geeignet sind, durch das Verfahren gemäß Anspruch 18 erhalten zu werden.

20. Verfahren zum Kontrollieren der Freisetzung eines Wirkstoffs und/oder Schutz vor einem Abbau, **dadurch gekennzeichnet, daß** es darin besteht, den Wirkstoff in eine lyotrope Phase einzuverleiben, die aus Tensiddoppelschichten gebildet ist, die Phase in ein erstes Medium zu dispergieren und das erste Medium in einem zweiten Medium zu emulgieren, das mit dem ersten Medium nicht mischbar ist.

21. Verwendung eines komplexen Mediums nach einem der Ansprüche 1 bis 14, um ein Medium auszuführen, das einen pH-Unterschied aufweist.

22. Verwendung eines komplexen Mediums nach einem der Ansprüche 1 bis 14, als Grundlage einer topischen Zusammensetzung zur kosmetischen oder dermathologischen Verwendung.

23. Verwendung eines komplexen Mediums nach Anspruch 15 zur Herstellung einer Zusammensetzung, die vorgesehen ist, die Immunantwort gegenüber dem Antigen zu verstärken.

24. Verwendung nach Anspruch 23, **dadurch gekennzeichnet, daß** die Doppellagen in Form von multilamellaren Vesikeln, sogenannten Vesikeln mit Zwiebelstruktur, im wesentlichen in Kugelform, sind, bestehend aus einer regelmäßigen Abfolge von konzentrischen Doppelschichten und dies vom Zentrum zur Peripherie der Vesikel hin.
